# EUROPEAN PATENT APPLICATION

(11) **EP 4 328 590 A1**
(43) Date of publication of application: **28.02.2024**
(21) Application number: 22791771.3
(22) Date of filing: 21.04.2022
(51) Int. Cl.: G01N 33/68, C12Q 1/37, C12Q 1/42, C12Q 1/44, C12Q 1/48, G01N 33/70, G01N 33/78, G01N 33/92, G16H 50/00

(54) **INFORMATION PROCESSING DEVICE FOR PROVIDING REFERENCE INFORMATION RELATING TO DIAGNOSIS OF THYROID DISEASE**

(30) Priority: 21.04.2021 JP 2021072021
(71) Applicant: Ito, Koichi, Tokyo 150-0001 (JP); F. Hoffmann-La Roche AG, 4070 Basel (CH); Roche Diagnostics GmbH, 68305 Mannheim (DE)
(72) Inventor: YOSHIMURA, Hiroshi, Tokyo 150-0001 (JP)
(74) Representative: HGF
(86) International application number: PCT/JP2022/018395
(87) International publication number: WO 2022/225000

(57) **Abstract**

An information processing device includes a first determination unit that inputs information related to total protein (TP), cholinesterase (ChE), total cholesterol (TC), creatinine (CREA), and creatine phosphokinase (CPK) to a first learning-completed model based on information related to a blood examination of a subject and outputs information related to whether thyroid stimulation hormone (TSH) of the subject is in a range for which medical treatment is needed.

## Description

### Technical Field

The present invention relates to an information processing device, a computer program, and an information processing method that provide reference information related to thyroid disease diagnosis.

### Background Art

Recently, it has been attempted to establish a model that predicts whether a subject is affected with a particular disease, has a risk of being affected with the disease, or the like based on genes, medical image data, or blood examinations, and utilize the model as auxiliary information for diagnosis by a doctor.

For example, Patent Literature 1 discloses a device that evaluates the risk of prostate cancer based on information of blood markers including a free prostate-specific antigen (fPSA) value and a total PSA (tPSA) value.

### Citation List

### Patent Literature

Patent Literature 1
Japanese Patent Laid-Open No. 2020-073885

### Summary of Invention

### Technical Problem

Main factors of thyrotoxicosis in Japan are, for example, Graves' disease (GD) and painless thyroiditis (PT). Diagnosis of Graves' disease (GD) is confirmed based on examination findings such as suppression of blood-serum free thyroxine (T4), free triiodothyronine (T3), and blood-serum thyroid stimulation hormone (TSH), existence of a thyroid stimulation antibody, and/or increase of radioactive iodine intake. Differentiation is sometimes made based on a condition that the amount of radioactive iodine intake is smaller for painless thyroiditis (PT) than for Graves' disease.

Diagnosis is delayed in some cases because symptoms of thyrotoxicosis are different among individuals and a thyroid function examination is not performed in a normal health checkup. For example, the risk of atrial fibrillation, congestive heart failure, and bone fracture becomes high when medical treatment of Graves' disease is delayed, and early medical treatment of Graves' disease is useful for avoiding severe cardiac complication or musculoskeletal complication.

The present invention is made in view of the above-described problem and is intended to provide an information processing device, a computer program, and an information processing method that are capable of providing reference information related to thyroid disease diagnosis based on biochemical parameters such as a result of a hematological examination performed in a normal health checkup, age, and weight.

### Solution to Problem

An information processing device according to a first embodiment of the present invention includes a first determination unit that inputs information related to total protein (TP), cholinesterase (ChE), total cholesterol (TC), creatinine (CREA), and creatine phosphokinase (CPK) to a first learning-completed model based on information related to a blood examination of a subject and outputs information related to whether thyroid stimulation hormone (TSH) of the subject is in a range for which medical treatment is needed.

With this configuration, primary information of whether the value of thyroid stimulation hormone (TSH) of a subject is in a range for which medical treatment is needed can be obtained based on a result of a hematological examination performed in a normal health checkup. The primary information can be used as reference information of whether the subject is affected with hypothyroidism or the degree of its advance. Note that the expression "thyroid stimulation hormone (TSH) is in a range for which medical treatment is needed" means that, for example, thyroid stimulation hormone (TSH) is equal to or more than 10 µIU/ml, but the range for which medical treatment is needed is not limited to this numerical range.

An information processing device according to a second embodiment of the present invention includes a second determination unit that inputs information related to total protein (TP), creatinine (CREA), neutrophil (Neu), and total bilirubin (T-Bill) to a second learning-completed model based on an age of a subject and information related to a blood examination of the subject and outputs information related to whether thyroid stimulation hormone (TSH) of the subject is in a normal value range.

With this configuration, primary information of whether thyroid stimulation hormone (TSH) of a subject is in a normal value range can be obtained based on a result of a hematological examination performed in a normal health checkup and the age of the subject. Note that the expression "thyroid stimulation hormone (TSH) is in a normal value range" means that, for example, thyroid stimulation hormone (TSH) is equal to or less than 4.5 µIU/ml, but the normal value range is not limited to this numerical range. For example, the normal value of thyroid stimulation hormone (TSH) may be set to be equal to or less than 5.0 µIU/ml.

An information processing device according to a third embodiment of the present invention includes a third determination unit that inputs information related to creatinine (CREA), total cholesterol (TC), alkaline phosphatase (ALP), and total protein (TP) to a third learning-completed model based on an age of a subject and information related to a blood examination of the subject and outputs information related to whether the subject is affected with Graves' disease.

With this configuration, primary information of whether a subject is affected with Graves' disease can be obtained based on a result of a hematological examination performed in a normal health checkup and the age of the subject. The primary information can be used as reference information of whether to perform an additional examination for determining Graves' disease and as reference information of the advance status of Graves' disease of the subject.

An information processing device according to a fourth embodiment of the present invention includes a fourth determination unit that inputs information related to thyroid hormone FT3, thyroid hormone FT4, FT3/FT4, alkaline phosphatase (ALP), and creatinine (CREA) to a fourth learning-completed model based on information related to a blood examination of a subject and outputs information related to differentiation of whether the subject is affected with Graves' disease or painless thyroiditis.

With this configuration, primary information on differentiation of whether a subject is affected with Graves' disease or painless thyroiditis can be obtained based on information of a result of a thyroid hormone examination in addition to a result of a hematological examination performed in a normal health checkup. The primary information can be used as reference information of whether to perform an additional examination for determining Graves' disease or painless thyroiditis.

An information processing device according to a fifth embodiment of the present invention includes a fifth determination unit that inputs information related to alkaline phosphatase (ALP), creatinine (CREA), total protein (TP), y glutamyl transpeptidase (yGTP), and white blood cells (WBC) to a fifth learning-completed model based on information related to a blood examination of a subject and outputs information related to differentiation of whether the subject is affected with Graves' disease or painless thyroiditis.

With this configuration, primary information on differentiation of whether a subject is affected with Graves' disease or painless thyroiditis can be obtained based on a result of a hematological examination performed in a normal health checkup irrespective of a result of a thyroid hormone examination. The primary information can be used as reference information of whether to perform an additional examination for determining Graves' disease or painless thyroiditis.

An information processing device according to a sixth embodiment of the present invention includes a sixth determination unit that inputs information related to total cholesterol (TC), cholinesterase (ChE), creatinine (CREA), and creatine phosphokinase (CPK) to a sixth learning-completed model based on an age of a subject and information related to a blood examination of the subject and outputs information related to whether the subject is affected with painless thyroiditis.

With this configuration, primary information of whether a subject is affected with painless thyroiditis can be obtained based on a result of a hematological examination performed in a normal health checkup. The primary information can be used as reference information of whether to perform an additional examination for determining painless thyroiditis and as reference information of the advance status of painless thyroiditis of the subject.

An information processing device according to a seventh embodiment of the present invention includes a seventh determination unit that inputs information related to creatinine (CREA), total cholesterol (TC), cholinesterase (ChE), creatine phosphokinase (CPK), and basophil (Ba) to a seventh learning-completed model based on information related to a blood examination of a subject and outputs information related to whether the subject is affected with thyrotoxicosis.

With this configuration, primary information of whether a subject is affected with thyrotoxicosis can be obtained based on a result of a hematological examination performed in a normal health checkup. The primary information can be used as reference information on whether to perform an additional examination for determining details of thyrotoxicosis and as reference information on the advance status of thyrotoxicosis of the subject.

The present invention also provides a computer program for causing an information processing device to execute the information inputting to and outputting from the learning-completed models in the above-described first and seventh embodiments, and an information processing method by which an information processing device executes the information inputting to and outputting from the learning-completed models.

### Advantageous Effects of Invention

According to the present invention, it is possible to provide an information processing device, a computer program, and an information processing method that are capable of providing reference information related to thyroid disease diagnosis based on biochemical parameters such as a result of a hematological examination performed in a normal health checkup and an age.

### Brief Description of Drawings

[Figure 1] Figure 1 is a block diagram illustrating an information processing device 100 included in a diagnosis system of the present embodiment.
[Figure 2] Figure 2 is an example of a flowchart exemplarily illustrating machine learning processing performed by a learning unit.
[Figure 3] Figure 3 is an example of a flowchart exemplarily illustrating processing performed by a determination unit.
[Figure 4] Figure 4 illustrates an example of a processing sequence of a diagnosis system constituted by a server and a user terminal.

### Description of Embodiments

Each embodiment of the present invention (hereinafter referred to as "the present embodiment") will be described below in detail, but the present invention is not limited thereto and may include various modifications without departing from the scope of the invention.

First, definitions of terms in the present embodiment will be described below.

"Hypothyroidism" refers to a state in which thyroid hormone action in an organ is less than needed. Typical symptoms of hypothyroidism include apathy, fatigue, swollenness, coldness, weight increase, slow movement, memory deterioration, and constipation, and mild hypothyroidism often hardly has symptoms and findings. Severe hypothyroidism causes somnolence and impaired consciousness and is called myxedema coma in some cases.

"Thyrotoxicosis" refers to a state in which thyroid hormone action in an organ is excessive. In thyrotoxicosis, metabolism is active and hotness and excessive sweating occur. Since energy is wastefully consumed, the size of a meal increases but weight decreases in some cases. Furthermore, nerves are affected and symptoms such as hand tremor, irritation, and diarrhea appear at various body sites. Thyrotoxicosis includes Graves' disease and painless thyroiditis as follows.

"Graves' disease" refers to a disease that thyroid hormone is excessively produced. Specifically, Graves' disease refers to a disease that thyroid hormone is excessively produced due to thyroid stimulation by a TSH receptor antibody.

"Painless thyroiditis" refers to a state in which the amount of thyroid hormone is temporarily increased due to leakage of thyroid hormone stored in thyroid into blood for some reasons. Specifically, thyroid hormone leaks into blood when a thyroid follicle breaks.

In the present embodiment, each disease typically means at least one of a disease diagnosed in accordance with guide lines published by a medical society related to the disease, a disease written in an effect-efficacy section on a package leaflet of a medical product, and a disease understood as a term generally used in medical drug and medical fields.

"Information related to whether" or "information related to differentiation" may be, for example, information that a subject is healthy or normal or information that expresses the probability that the subject is healthy or normal in percentage or at a normalized level. Alternatively, the information may be information that a subject is affected with a predetermined disease or information that expresses the probability that the subject is affected with the predetermined disease in percentage or at a normalized level. The information may include both information that the subject is normal or healthy and information that the subject is affected with a disease. In place of or in addition to this, the information may include qualitative or quantitative information that, for example, a biochemical parameter related to a disease is higher (for example, closer to being affected) or lower (for example, closer to being normal or healthy) than a reference value for determination of a subject being normal or healthy. Moreover, the information may include a meaning of recommending a further examination such as a thorough examination.

### 1. First embodiment

### 1.1. Hardware configuration

In a first embodiment, a diagnosis system 1 that determines whether thyroid stimulation hormone (TSH) of a subject is in a range for which medical treatment is needed based on information related to total protein (TP), cholinesterase (ChE), total cholesterol (TC), creatinine (CREA), and creatine phosphokinase (CPK) is established by an information processing device 100 that is a thyroid disease determination device.

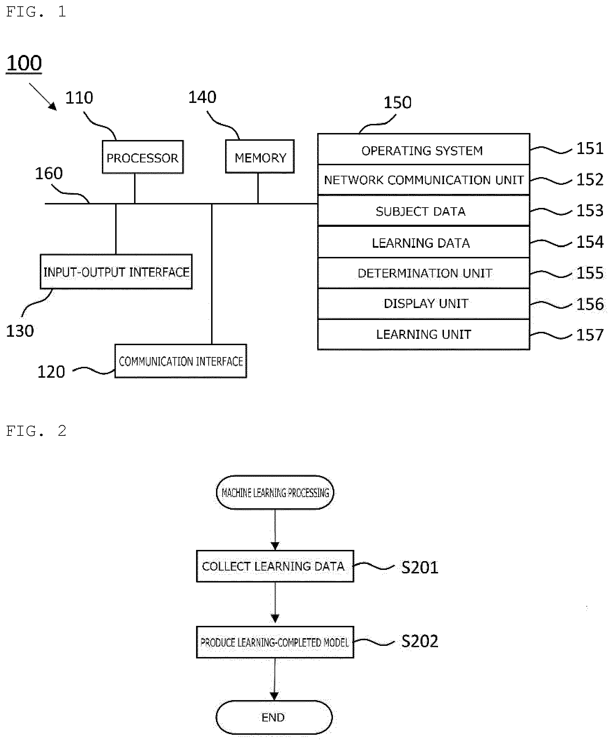

Figure 1 is a block diagram illustrating the information processing device 100 included in the diagnosis system of the present embodiment. The information processing device 100 typically includes one or a plurality of processors 110, a communication interface 120 configured to control wired or wireless communication, an input-output interface 130, a memory 140, a storage 150, and one or a plurality of communication buses 160 for mutually connecting these constituent components, and achieves processing, functions, or methods described in the present disclosure through cooperation of them.

Each processor 110 executes processing, a function, or a method implemented by a code included in a computer program stored in the memory 140 or by a command. The processor 110 includes, not as a limitation but as an example, one or a plurality of central processing units (CPUs) and graphics processing units (GPUs).

The communication interface 120 transmits and receives various kinds of data to and from another information processing device through a network. This communication may be executed in any of wired and wireless manners, and any communication protocol may be used as long as communication can be executed therebetween. For example, the communication interface 120 is implemented as a hardware component such as a network adapter, a communication software component of any kind, or a combination thereof.

The input-output interface 130 includes an input device for inputting various operations to the information processing device 100, and an output device for outputting results of processing executed by the information processing device 100. For example, the input-output interface 130 includes an information input device such as a keyboard, a mouse, or a touch panel, a blood examination device, and an image output device such as a display. Note that the information processing device 100 may be connected to the input-output interface 130 outside to receive predetermined inputting. For example, the information processing device 100 may be connected to an external blood examination device.

The memory 140 temporarily stores a computer program loaded from the storage 150 and provides a work area for the processor 110. The memory 140 also temporarily stores various kinds of data generated while the processor 110 executes the computer program. The memory 140 may be, not as a limitation but as an example, a high-speed random access memory such as a DRAM, an SRAM, a DDR RAM, or another random access solid storage device or may be a combination thereof.

The storage 150 stores computer programs, various kinds of functional units, and various kinds of data. The storage 150 may be, not as a limitation but as an example, a magnetic disk storage device, an optical disk storage device, a flash memory device, or another non-volatile memory such as a non-volatile solid storage device or may be a combination thereof. Alternatively, the storage 150 may be, for example, one or a plurality of storage devices installed remotely from a CPU 110.

In an embodiment of the present invention, the storage 150 stores a computer program, a functional unit, a data structure, or a subset thereof. In the example illustrated in Figure 1, the information processing device 100 is configured to function as a determination unit 155, a display unit 156, and a learning unit 157 as the processor 110 executes commands included in a computer program stored in the storage 150.

An operating system 151 includes, for example, procedures for processing various basic system services and executing tasks by using hardware.

A network communication unit 152 is used to, for example, connect the information processing device 100 to another computer through the communication interface 120 and one or a plurality of communication networks such as the Internet, another wide area network, a local area network, and a metropolitan area network.

Subject data 153 includes, for example, private information of a subject such as sex, age, and blood examination results. The subject data 153 may be an electronic medical record stored in the storage 150 or an electronic medical record stored in a remote server.

Learning data 154 is a data set used to generate a learning-completed model, and the data set may be used separately as teacher data and test data. The learning data 154 includes, for example, information related to thyroid disease and thyroid stimulation hormone and information related to a blood examinations. The learning data 154 may be stored in the storage 150 or a remote server.

The determination unit 155 executes processing that acquires information related to a hematological examination of a subject from the communication interface 120 or the input-output interface 130. For example, the information acquired by the determination unit 155 can be input to the information processing device 100 through the communication interface 120 by a doctor, a nurse, any other medical person, or a medical institution. Alternatively, the information acquired by the determination unit 155 may be received by the determination unit 155 from another terminal or server through the input-output interface 130. The determination unit 155 can store the acquired information in the subject data 153.

Hereinafter, a determination unit used in the first embodiment is referred to as a first determination unit 155, and determination units used in second to sixth embodiments to be described later are referred to as a second to sixth determination units 155, respectively. When not needing to be distinguished from one another, these determination units are simply referred to as the determination unit 155. Similarly, learning-completed models used in the first to sixth embodiments are referred to as first to second learning-completed models, respectively, and simply referred to as a learning-completed model when not needing to be distinguished from one another.

Information related to a hematological examination and acquired by the first determination unit 155 includes, for example, total protein (TP), cholinesterase (ChE), total cholesterol (TC), creatinine (CREA), and creatine phosphokinase (CPK) of a subject and may include other information.

The first determination unit 155 executes processing that inputs information related to a hematological examination of a subject and acquired as described above to the first learning-completed model and outputs information related to whether thyroid stimulation hormone (TSH) of the subject is in a range for which medical treatment is needed. The first learning-completed model is not particularly limited but may be, for example, a learning-completed model generated by machine learning processing based on the above-described learning data 154.

A display unit 157 executes processing that controls display of the above-described information output from the first determination unit 155 on a display device. For example, the display unit 157 can control display of the information related to whether thyroid stimulation hormone (TSH) of a subject is in a range for which medical treatment is needed on the display device as the probability that thyroid stimulation hormone (TSH) of the subject is equal to or more than 10 µIU/ml or the probability that thyroid stimulation hormone (TSH) of the subject is less than 10 µIU/ml.

A learning unit 158 executes processing that generates a learning-completed model through machine learning processing based on the learning data 154. Examples of the machine learning processing include supervised learning and semi-supervised learning.

### 1.2. Learning-completed model

As an example, the first learning-completed model was produced by using Prediction One manufactured by Sony Network Communications Inc. The production of the first learning-completed model was performed by using learning data of 3343 test subjects the value of thyroid stimulation hormone (TSH) of which is more than 10 µIU/ml and 23716 test subjects the value of thyroid stimulation hormone (TSH) of which is equal to or less than 10 µIU/ml.

Specifically, a data set of 18939 examples of teacher data and 8120 examples of test data was prepared from the learning data and the production of the first learning-completed model was performed by using the teacher data. As a result, the first learning-completed model that uses total protein (TP), cholinesterase (ChE), total cholesterol (TC), creatinine (CREA), and creatine phosphokinase (CPK) as prediction factors was obtained.

Subsequently, a receiver operating characteristic (ROC) curve of the first learning-completed model and the area under the curve (AUC) were calculated based on the above-described test data, and the following evaluation result of the first learning-completed model was obtained.
AUC 0.737

Note that the data of each example may include the value of thyroid stimulation hormone (TSH), a hematological examination result, and the age, sex, and weight of a test subject. The hematological examination result includes red blood count (RBC), the amount of hemoglobin (Hb), hematocrit (Ht), mean corpuscular hemoglobin volume (MCV), mean corpuscular hemoglobin (MCH), mean corpuscular hemoglobin concentration (MCHC), platelet count (Plt), white blood count (WBC), neutrophil (Neu), lymphocyte (Lym), monocyte (Mo), eosinophil (Eo), basophil (Ba), total protein (TP), total bilirubin (T-Bil), aspartate aminotransferase (AST), alanine aminotransferase (ALT), lactate dehydrogenase (LDH), y glutamyl transpeptidase (yGTP), alkaline phosphatase (ALP), cholinesterase (ChE), creatine phosphokinase (CPK), creatinine (CREA), uric acid (UA), Na, K, Cl, Ca, P, and total cholesterol (TC). In addition, the data of each example may include information related to change of each value of the hematological examination result over time and change of the weight over time. This also applies to other aspects described below.

### 1.2.1. Learning-completed model (modification 1)

As another example, the first learning-completed model was produced by using learning data of 2427 test subjects the value of thyroid stimulation hormone (TSH) of which is more than 15 µIU/ml and 24632 test subjects the value of thyroid stimulation hormone (TSH) of which is equal to or less than 15 µIU/ml.

Specifically, the learning data was divided at the ratio of 7:3 to prepare a data set of teacher data and test data, and the production of the first learning-completed model was performed by using the teacher data. As a result, the first learning-completed model that uses total protein (TP), cholinesterase (ChE), total cholesterol (TC), creatinine (CREA), and creatine phosphokinase (CPK) as prediction factors like the above-described model was obtained.

Subsequently, the ROC curve and AUC of the first learning-completed model were calculated by using the above-described test data. The following evaluation result of the first learning-completed model was obtained.
AUC 0.773

As described above, the first learning-completed model obtained in this manner has specificity, correct diagnosis percentage, and negativity correctness percentage of 90% or higher, and it is understood that approximately half of patients having high thyroid stimulation hormone (TSH) in a range for which medical treatment is needed can be detected with a reduced number of unnecessary examinations.

### 1.3. Operation processing

Operation of the information processing device 100 of the first embodiment thus configured will be described below.

### 1.3.1. Production of learning-completed model

Figure 2 is a flowchart exemplarily illustrating machine learning processing through which the learning unit 158 of the information processing device 100 produces a learning-completed model by using teacher data.

At step S201, the learning unit 158 collects actual medical treatment data as learning data. In this case, the learning unit 158 collects actual medical treatment data from among the subject data 153 of the information processing device 100 or subject data stored in another information processing device or server connected through a network and stores the actual medical treatment data in the learning data 154. The actual medical treatment data can be used as teacher data and test data.

At step S202, the learning unit 158 produces teacher data from a data set stored in the learning data 154 and produces, by using the teacher data, the first learning-completed model that outputs information related to whether thyroid stimulation hormone (TSH) is in a range for which medical treatment is needed. For example, the learning unit 158 performs machine learning by using the teacher data with a correct answer label set to information related to the value of thyroid stimulation hormone (TSH).

### 1.3.2. Determination using learning-completed model

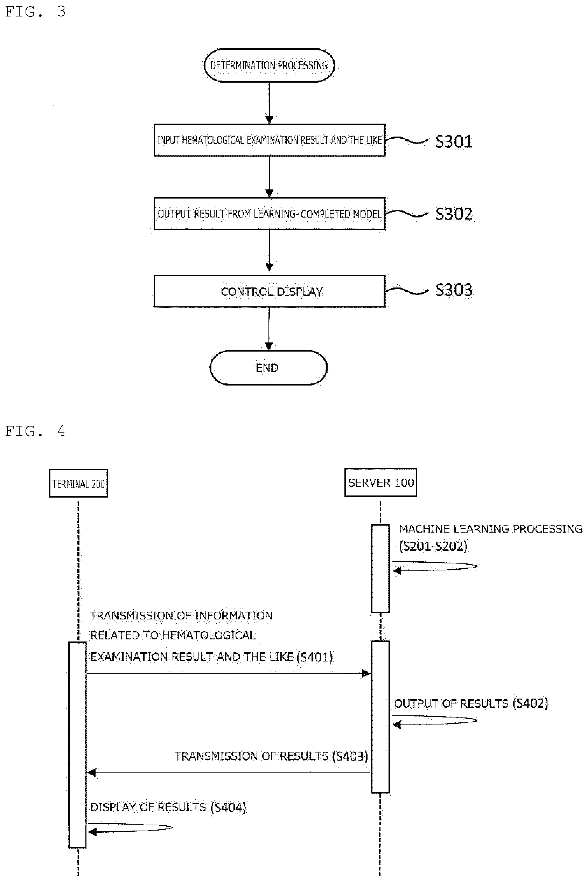

Figure 3 is a flowchart exemplarily illustrating processing that the determination unit 155 of the information processing device 100 outputs, by using a learning-completed model, information related to visual prognosis after medical treatment is performed.

At step S301, the determination unit 155 acquires information related to a hematological examination result of a subject. In this case, the determination unit 155 may acquire information related to a hematological examination result from another information processing device or a measurement device through the communication interface 120 or may acquire a hematological examination result from an information input device such as a keyboard or a touch panel through the input-output interface 130. For example, the determination unit 155 may acquire information related to medical treatment to be performed, such as disease names or symptoms of the subject, from electronic medical record information stored in the storage of the information processing device or a server accessible from the information processing device in place of an input operation by a doctor.

At step S302, the determination unit 155 inputs the information related to a hematological examination result of the subject to the first learning-completed model and outputs, from the first learning-completed model, information related to whether thyroid stimulation hormone (TSH) is in a range for which medical treatment is needed.

At step S303, the display unit 156 controls display of the output information on an image output device such as a display. The display control is not limited to a particular method, but for example, may indicate, in percentage, the probability that thyroid stimulation hormone (TSH) is in a range for which medical treatment is needed and the probability that thyroid stimulation hormone (TSH) is not in the range for which medical treatment is needed.

With the diagnosis system provided by the information processing device 100 according to the first embodiment described above, for example, primary information of whether the value of thyroid stimulation hormone (TSH) of a subject is in a range for which medical treatment is needed can be obtained based on a result of a hematological examination performed in a normal health checkup.

### 2. Second embodiment

In the second embodiment, the diagnosis system 1 that determines whether thyroid stimulation hormone (TSH) of a subject is in a normal value range based on the age of the subject and information related to total protein (TP), creatinine (CREA), neutrophil (Neu), and total bilirubin (T-Bill) from information related to a blood examination of the subject is established by the information processing device 100 that is a thyroid disease determination device.

The configuration, operation processing, and the like of the information processing device 100 according to the second embodiment are basically the same as the configuration, operation processing, and the like of the information processing device of the diagnosis system according to the first embodiment. The second embodiment is different from the first embodiment in that information input to the second learning-completed model includes the age of a subject and information related to total protein (TP), creatinine (CREA), neutrophil (Neu), and total bilirubin (T-Bill) as information related to a blood examination of the subject, and in that information output from the second learning-completed model is information related to whether thyroid stimulation hormone (TSH) of the subject is in a normal value range.

In the following description of the second embodiment, the same functional configurations and steps as in the first embodiment are denoted by the same reference signs as in the first embodiment and description thereof is omitted, and the difference of the second embodiment from the first embodiment will be mainly described.

### 2.1. Hardware configuration

Information related to a hematological examination and acquired by the second determination unit 155 includes, for example, total protein (TP), creatinine (CREA), neutrophil (Neu), and total bilirubin (T-Bill) of a subject and may include other information. Biochemical parameters acquired by the second determination unit 155 include the age of the subject and may include other information.

The second determination unit 155 executes processing that inputs the age and information related to a hematological examination of the subject, which are acquired as described above, to the second learning-completed model and outputs information related to whether thyroid stimulation hormone (TSH) of the subject is in a normal value range. The second learning-completed model is not particularly limited but may be, for example, a learning-completed model generated through machine learning processing based on the above-described learning data 154.

The display unit 157 executes processing that controls display of the above-described information output from the second determination unit 155 on the display device. For example, the display unit 157 can control display of the information related to whether thyroid stimulation hormone (TSH) of the subject is in the normal value range on the display device as the probability that thyroid stimulation hormone (TSH) of the subject is equal to or less than 4.5 µIU/ml or the probability that thyroid stimulation hormone (TSH) of the subject is more than 4.5 µIU/ml.

The learning unit 158 executes processing that generates a learning-completed model through which machine learning processing based on the learning data 154. Examples of the machine learning processing include supervised learning and semi-supervised learning.

### 2.2. Learning-completed model

As an example, the second learning-completed model was produced by using Prediction One manufactured by Sony Network Communications Inc. The production of the second learning-completed model was performed by using learning data of 7850 test subjects the value of thyroid stimulation hormone (TSH) of which is more than 4.5 µIU/ml and 19209 test subjects the value of thyroid stimulation hormone (TSH) of which is 0.2 to 4.5 µIU/ml or less.

Specifically, the learning data was divided at the ratio of 7:3 to prepare a data set of teacher data and test data, and the production of the second learning-completed model was performed by using the teacher data. As a result, the second learning-completed model that uses total protein (TP), age, creatinine (CREA), neutrophil (Neu), and total bilirubin (T-Bill) as prediction factors was obtained.

Subsequently, the ROC curve and AUC of the second learning-completed model were calculated by using the above-described test data. The following evaluation result of the second learning-completed model was obtained.
AUC 0.639

### 2.3. Operation processing

Operation of the information processing device 100 of the second embodiment thus configured will be described below.

At step S202, the learning unit 158 produces teacher data from a data set stored in the learning data 154 and produces, by using the teacher data, the second learning-completed model that outputs information related to whether thyroid stimulation hormone (TSH) is in a normal value range. For example, the learning unit 158 performs machine learning by using the teacher data with a correct answer label set to information related to the value of thyroid stimulation hormone (TSH).

At step S302, the determination unit 155 inputs information related to a hematological examination result of a subject to the learning-completed model and outputs, from the learning-completed model, information related to whether thyroid stimulation hormone (TSH) is in the normal value range.

At step S303, the display unit 156 controls display of the output information on an image output device such as a display. The display control is not limited to a particular method, but for example, may indicate, in percentage, the probability that thyroid stimulation hormone (TSH) is in the normal value range and the probability that thyroid stimulation hormone (TSH) is not in the normal value range.

### 3. Third embodiment

In the third embodiment, the diagnosis system 1 that determines whether a subject is affected with Graves' disease based on the age of the subject and information related to creatinine (CREA), total cholesterol (TC), alkaline phosphatase (ALP), and total protein (TP) from information related to a blood examination of the subject is established by the information processing device 100 that is a thyroid disease determination device.

The configuration, operation processing, and the like of the information processing device 100 according to the third embodiment are basically the same as the configuration, operation processing, and the like of the information processing device of the diagnosis system according to the first embodiment. The third embodiment is different from the first embodiment in that information input to a third learning-completed model includes the age of a subject and information related to creatinine (CREA), total cholesterol (TC), alkaline phosphatase (ALP), and total protein (TP) as information related to a blood examination of the subject, and in that information output from the third learning-completed model is information related to whether the subject is affected with Graves' disease.

In the following description of the third embodiment, the same functional configurations and effects as in the first embodiment are denoted by the same reference signs as in the first embodiment and description thereof is omitted, and the difference of the third embodiment from the first embodiment will be mainly described.

### 3.1. Hardware configuration

Information related to a hematological examination and acquired by the third determination unit 155 includes, for example, creatinine (CREA), total cholesterol (TC), alkaline phosphatase (ALP), and total protein (TP) of a subject and may include other information. Biochemical parameters acquired by the third determination unit 155 include the age of the subject and may include other information.

The third determination unit 155 executes processing that inputs the age and information related to a hematological examination of the subject, which are acquired as described above, to the third learning-completed model, and outputs information related to whether the subject is affected with Graves' disease. The third learning-completed model is not particularly limited but may be, for example, a learning-completed model generated through machine learning processing based on the above-described learning data 154.

The display unit 157 executes processing that controls display of the above-described information output from the third determination unit 155 on the display device. For example, the display unit 157 can control display of the information related to whether the subject is affected with Graves' disease on the display device as the probability that the subject is affected with Graves' disease or the probability that the subject is not affected with Graves' disease.

The learning unit 158 executes processing that generates a learning-completed model through machine learning processing based on the learning data 154. Examples of the machine learning processing include supervised learning and semi-supervised learning.

### 3.2. Learning-completed model

As an example, the third learning-completed model was produced by using Prediction One manufactured by Sony Network Communications Inc. The production of the third learning-completed model was performed by using learning data of 19335 untreated Graves' disease patients and 4159 healthy subjects.

Specifically, the learning data was divided at the ratio of 7:3 to prepare a data set of teacher data and test data, and the production of the third learning-completed model was performed by using the teacher data. As a result, the third learning-completed model that uses creatinine (CREA), total cholesterol (TC), alkaline phosphatase (ALP), age, and total protein (TP) of a subject as prediction factors was obtained.

Subsequently, the ROC curve and AUC of the third learning-completed model were calculated by using the above-described test data. The following evaluation result of the third learning-completed model was obtained.
AUC 0.974

### 3.2.1. Learning-completed model (modification 1)

In addition, in order to check dependency on sex for determination of whether a subject is affected with Graves' disease, the above-described learning data was divided into data of male and data of female to prepare a data set of teacher data and test data, and production of the third learning-completed model (female) and the third learning-completed model (male) was performed by using the teacher data.

As a result, a model based on prediction factors of creatinine (CREA), total cholesterol (TC), cholinesterase (ChE), alkaline phosphatase (ALP), and creatine phosphokinase (CPK) was obtained as the third learning-completed model (female), and a model based on prediction factors of alkaline phosphatase (ALP), creatinine (CREA), total cholesterol (TC), total protein (TP), and alanine aminotransferase (ALT) was obtained as the third learning-completed model (male).

The ROC curve and AUC of each of the third learning-completed model (female) and the third learning-completed model (male) obtained in this manner were calculated by using the above-described test data.

The third learning-completed model of the third embodiment may be replaced with the third learning-completed model (female) or the third learning-completed model (male) when used.

### 3.2.2. Learning-completed model (modification 2)

In addition, in order to check dependency on a free thyroxine (FT4) level for determination of whether a subject is affected with Graves' disease, test data for producing the third learning-completed model (severe GD) that determines severe thyrotoxicosis with the free thyroxine (FT4) level of 5 ng/dl or higher and test data for producing the third learning-completed model (mild GD) that determines mild thyrotoxicosis with the free thyroxine (FT4) level of 5 ng/dl or less were produced from the above-described learning data.

The test data for producing the third learning-completed model (severe GD) includes teacher data including 6040 severe thyrotoxicosis patients with the free thyroxine (FT4) level of 5 ng/dl or higher and 2911 healthy subjects, and test data of 2516 randomly selected thyrotoxicosis patients and 1248 healthy subjects.

The test data for producing the third learning-completed model (mild GD) includes teacher data including mild 7505 thyrotoxicosis patients with the free thyroxine (FT4) level of 5 ng/dl or less and 2911 healthy subjects, and test data of 3275 randomly selected thyrotoxicosis patients and 1248 healthy subjects.

As a result, a model based on prediction factors of total cholesterol (TC), creatinine (CREA), total protein (TP), cholinesterase (ChE), and monocyte (Mo) was obtained as the third learning-completed model (severe GD), and a model based on prediction factors of creatinine (CREA), total cholesterol (TC), age, alkaline phosphatase (ALP), and total protein (TP) was obtained as the third learning-completed model (mild GD).

The ROC curve and AUC of each of the third learning-completed model (severe GD) and the third learning-completed model (mild GD) obtained in this manner were calculated by using the above-described test data.

The third learning-completed model of the third embodiment may be replaced with the third learning-completed model (severe GD) or the third learning-completed model (mild GD) when used.

### 3.3. Operation processing

Operation of the information processing device 100 of the third embodiment thus configured will be described below.

At step S202, the learning unit 158 produces teacher data from a data set stored in the learning data 154 and produces, by using the teacher data, the third learning-completed model that outputs information related to whether a subject is affected with Graves' disease. For example, the learning unit 158 performs machine learning by using the teacher data with a correct answer label set to information related to whether the subject is affected with Graves' disease.

At step S302, the determination unit 155 inputs information related to a hematological examination result of the subject to the third learning-completed model and outputs, from the third learning-completed model, information related to whether the subject is affected with Graves' disease.

At step S303, the display unit 156 controls display of the output information on an image output device such as a display. The display control is not limited to a particular method, but for example, may indicate, in percentage, the probability that the subject is affected with Graves' disease and the probability that the subject is not affected with Graves' disease.

### 4. Fourth embodiment

In the fourth embodiment, the diagnosis system 1 that inputs information related to thyroid hormone FT3, thyroid hormone FT4, FT3/FT4, alkaline phosphatase (ALP), and creatinine (CREA) from information related to a blood examination of a subject to a fourth learning-completed model and differentiates whether the subject is affected with Graves' disease or painless thyroiditis is established by the information processing device 100 that is a thyroid disease determination device.

The configuration, operation processing, and the like of the information processing device 100 according to the fourth embodiment are basically the same as the configuration, operation processing, and the like of the information processing device of the diagnosis system according to the first embodiment. The fourth embodiment is different from the first embodiment in that information input to the fourth learning-completed model includes information related to thyroid hormone FT3, thyroid hormone FT4, FT3/FT4, alkaline phosphatase (ALP), and creatinine (CREA) as information related to a blood examination of a subject, and in that information output from the fourth learning-completed model is information related to differentiation of whether the subject is affected with Graves' disease or painless thyroiditis.

In the following description of the fourth embodiment, the same functional configurations and effects as in the first embodiment are denoted by the same reference signs as in the first embodiment and description thereof is omitted, and the difference of the fourth embodiment from the first embodiment will be mainly described.

### 4.1. Hardware configuration

Information related to a hematological examination and acquired by the fourth determination unit 155 includes, for example, thyroid hormone FT3, thyroid hormone FT4, FT3/FT4, alkaline phosphatase (ALP), and creatinine (CREA) of a subject and may include other information.

The fourth determination unit 155 executes processing that inputs the age and information related to a hematological examination of the subject, which are acquired as described above, to the fourth learning-completed model and outputs information related to differentiation of whether the subject is affected with Graves' disease or painless thyroiditis. The fourth learning-completed model is not particularly limited but may be, for example, a learning-completed model generated through machine learning processing based on the above-described learning data 154.

The display unit 157 executes processing that controls display of the above-described information output from the fourth determination unit 155 on the display device. For example, the display unit 157 can control display of the information related to differentiation of whether the subject is affected with Graves' disease or painless thyroiditis on the display device as the probability that the subject is affected with Graves' disease or the probability that the subject is not affected with Graves' disease, or as the probability that the subject is affected with painless thyroiditis or the probability that the subject is affected with painless thyroiditis.

The learning unit 158 executes processing that generates a learning-completed model through machine learning processing based on the learning data 154. Examples of the machine learning processing include supervised learning and semi-supervised learning.

### 4.2. Learning-completed model

As an example, the fourth learning-completed model was produced by using Prediction One manufactured by Sony Network Communications Inc. The production of the fourth learning-completed model was performed by using learning data of 19335 untreated Graves' disease patients and 3267 untreated painless thyroiditis patients.

Specifically, the learning data was divided at the ratio of 7:3 to prepare a data set of teacher data and test data, and the production of the fourth learning-completed model was performed by using the teacher data. As a result, the fourth learning-completed model that uses thyroid hormone FT3, thyroid hormone FT4, FT3/FT4, alkaline phosphatase (ALP), and creatinine (CREA) as prediction factors was obtained.

Subsequently, the ROC curve and AUC of the fourth learning-completed model were calculated by using the above-described test data.

### 4.3. Operation processing

Operation of the information processing device 100 of the fourth embodiment thus configured will be described below.

At step S202, the learning unit 158 produces teacher data from a data set stored in the learning data 154 and produces, by using the teacher data, the fourth learning-completed model that outputs information related to differentiation of whether a subject is affected with Graves' disease or painless thyroiditis. For example, the learning unit 158 performs machine learning by using the teacher data with a correct answer label set to information related to the disease (Graves' disease or painless thyroiditis) with which the subject is affected.

At step S302, the determination unit 155 inputs information related to a hematological examination result of the subject to the fourth learning-completed model and outputs, from the fourth learning-completed model, information related to differentiation of whether the subject is affected with Graves' disease or painless thyroiditis.

At step S303, the display unit 156 controls display of the output information on an image output device such as a display. The display control is not limited to a particular method, but for example, may indicate, in percentage, the probability that the subject is affected with Graves' disease and the probability that the subject is not affected with Graves' disease, or the probability that the subject is affected with painless thyroiditis and the probability that the subject is not affected with painless thyroiditis.

### 5. Fifth embodiment

In the fifth embodiment, the diagnosis system 1 that inputs information related to alkaline phosphatase (ALP), creatinine (CREA), total protein (TP), y glutamyl transpeptidase (yGTP), and white blood cells (WBC) from information related to a blood examination of a subject to a fifth learning-completed model and differentiates whether the subject is affected with Graves' disease or painless thyroiditis is established by the information processing device 100 that is a thyroid disease determination device.

The configuration, operation processing, and the like of the information processing device 100 according to the fifth embodiment are basically the same as the configuration, operation processing, and the like of the information processing device of the diagnosis system according to the first embodiment. The fifth embodiment is different from the first embodiment in that information input to the fifth learning-completed model includes information related to alkaline phosphatase (ALP), creatinine (CREA), total protein (TP), y glutamyl transpeptidase (yGTP), and white blood cells (WBC) as information related to a blood examination of the subject, and in that information output from the fifth learning-completed model is information related to differentiation of whether the subject is affected with Graves' disease or painless thyroiditis.

In the following description of the fifth embodiment, the same functional configurations and effects as in the first embodiment are denoted by the same reference signs as in the first embodiment and description thereof is omitted, and the difference of the fifth embodiment from the first embodiment will be mainly described.

### 5.1. Hardware configuration

Information related to a hematological examination and acquired by the fifth determination unit 155 includes, for example, alkaline phosphatase (ALP), creatinine (CREA), total protein (TP), y glutamyl transpeptidase (yGTP), and white blood cells (WBC) of a subject and may include other information.

The fifth determination unit 155 executes processing that inputs the age and information related to a hematological examination of the subject, which are acquired as described above, to the fifth learning-completed model and outputs information related to differentiation of whether the subject is affected with Graves' disease or painless thyroiditis. The fifth learning-completed model is not particularly limited but may be, for example, a learning-completed model generated through machine learning processing based on the above-described learning data 154.

The display unit 157 executes processing that controls display of the above-described information output from the fifth determination unit 155 on the display device. For example, the display unit 157 can control display of the information related to differentiation of whether the subject is affected with Graves' disease or painless thyroiditis on the display device as the probability that the subject is affected with Graves' disease or the probability that the subject is not affected with Graves' disease, or as the probability that the subject is affected with painless thyroiditis or the probability that the subject is not affected with painless thyroiditis.

The learning unit 158 executes processing that generates a learning-completed model through machine learning processing based on the learning data 154. Examples of the machine learning processing include supervised learning and semi-supervised learning.

### 5.2. Learning-completed model

As an example, the fifth learning-completed model was produced by using Prediction One manufactured by Sony Network Communications Inc. The production of the fifth learning-completed model was performed by using learning data of 19335 untreated Graves' disease patients and 3267 untreated painless thyroiditis patients.

Specifically, the learning data was divided at the ratio of 7:3 to prepare a data set of teacher data and test data, and the production of the fifth learning-completed model was performed by using the teacher data. As a result, the fifth learning-completed model that uses alkaline phosphatase (ALP), creatinine (CREA), total protein (TP), y glutamyl transpeptidase (yGTP), and white blood cells (WBC) as prediction factors was obtained.

Subsequently, the ROC curve and AUC of the fifth learning-completed model were calculated by using the above-described test data.

### 5.3. Operation processing

Operation of the information processing device 100 of the fifth embodiment thus configured will be described below.

At step S202, the learning unit 158 produces teacher data from a data set stored in the learning data 154 and produces, by using the teacher data, the fifth learning-completed model that outputs information related to differentiation of whether a subject is affected with Graves' disease or painless thyroiditis. For example, the learning unit 158 performs machine learning by using the teacher data with a correct answer label set to the information related to the disease (Graves' disease or painless thyroiditis) with which the subject is affected.

At step S302, the determination unit 155 inputs information related to a hematological examination result of the subject to the fifth learning-completed model and outputs, from the fifth learning-completed model, information related to differentiation of whether the subject is affected with Graves' disease or painless thyroiditis.

At step S303, the display unit 156 controls display of the output information on an image output device such as a display. The display control is not limited to a particular method, but for example, may indicate, in percentage, the probability that the subject is affected with Graves' disease and the probability that the subject is not affected with Graves' disease, or the probability that the subject is affected with painless thyroiditis and the probability that the subject is not affected with painless thyroiditis.

### 6. Sixth embodiment

In the sixth embodiment, the diagnosis system 1 that inputs the age of a subject and information related to total cholesterol (TC), cholinesterase (ChE), creatinine (CREA), and creatine phosphokinase (CPK) from information related to a blood examination of the subject to a sixth learning-completed model and determines whether the subject is affected with painless thyroiditis is established by the information processing device 100 that is a thyroid disease determination device.

The configuration, operation processing, and the like of the information processing device 100 according to the sixth embodiment are basically the same as the configuration, operation processing, and the like of the information processing device of the diagnosis system according to the first embodiment. The sixth embodiment is different from the first embodiment in that information input to the sixth learning-completed model includes the age of the subject and information related to total cholesterol (TC), cholinesterase (ChE), creatinine (CREA), and creatine phosphokinase (CPK) as information related to a blood examination of the subject, and in that information output from the sixth learning-completed model is information related to whether the subject is affected with painless thyroiditis.

In the following description of the sixth embodiment, the same functional configurations and effects as in the first embodiment are denoted by the same reference signs as in the first embodiment and description thereof is omitted, and the difference of the sixth embodiment from the first embodiment will be mainly described.

### 6.1. Hardware configuration

Information related to a hematological examination and acquired by the sixth determination unit 155 includes, for example, total cholesterol (TC), cholinesterase (ChE), creatinine (CREA), and creatine phosphokinase (CPK) of a subject and may include other information. Biochemical parameters acquired by the sixth determination unit 155 include the age of the subject and may include other information.

The sixth determination unit 155 executes processing that inputs the age and information related to a hematological examination of the subject, which are acquired as described above, to the sixth learning-completed model and outputs information related to whether the subject is affected with painless thyroiditis. The sixth learning-completed model is not particularly limited but may be, for example, a learning-completed model generated through machine learning processing based on the above-described learning data 154.

The display unit 157 executes processing that controls display of the above-described information output from the sixth determination unit 155 on the display device. For example, the display unit 157 can control display of the information related to whether the subject is affected with painless thyroiditis on the display device as the probability that the subject is affected with painless thyroiditis or the probability that the subject is not affected with painless thyroiditis.

The learning unit 158 executes processing that generates a learning-completed model through machine learning processing based on the learning data 154. Examples of the machine learning processing include supervised learning and semi-supervised learning.

### 6.2. Learning-completed model

As an example, the sixth learning-completed model was produced by using Prediction One manufactured by Sony Network Communications Inc. The production of the sixth learning-completed model was performed by using learning data of 3267 untreated painless thyroiditis patients and 4159 healthy subjects.

Specifically, the learning data was divided at the ratio of 7:3 to prepare a data set of teacher data and test data, and the production of the sixth learning-completed model was performed by using the teacher data. As a result, the sixth learning-completed model that uses total cholesterol (TC), cholinesterase (ChE), creatinine (CREA), age, and creatine phosphokinase (CPK) as prediction factors was obtained.

Subsequently, the ROC curve and AUC of the sixth learning-completed model were calculated by using the above-described test data.

### 6.3. Operation processing

Operation of the information processing device 100 of the sixth embodiment thus configured will be described below.

At step S202, the learning unit 158 produces teacher data from a data set stored in the learning data 154 and produces, by using the teacher data, the sixth learning-completed model that outputs information related to whether a subject is affected with painless thyroiditis. For example, the learning unit 158 performs machine learning by using the teacher data with a correct answer label set to information related to whether the subject is affected with painless thyroiditis.

At step S302, the determination unit 155 inputs information related to a hematological examination result of the subject to the sixth learning-completed model and outputs, from the sixth learning-completed model, information related to whether the subject is affected with painless thyroiditis.

At step S303, the display unit 156 controls display of the output information on an image output device such as a display. The display control is not limited to a particular method, but for example, may indicate, in percentage, the probability that the subject is affected with painless thyroiditis and the probability that the subject is not affected with painless thyroiditis.

### 7. Seventh embodiment

In the seventh embodiment, the diagnosis system 1 that inputs information related to creatinine (CREA), total cholesterol (TC), cholinesterase (ChE), creatine phosphokinase (CPK), and basophil (Ba) from information related to a blood examination of a subject to a seventh learning-completed model and determines whether the subject is affected with thyrotoxicosis is established by the information processing device 100 that is a thyroid disease determination device.

The configuration, operation processing, and the like of the information processing device 100 according to the seventh embodiment are basically the same as the configuration, operation processing, and the like of the information processing device of the diagnosis system according to the first embodiment. The seventh embodiment is different from the first embodiment in that information input to the seventh learning-completed model includes information related to creatinine (CREA), total cholesterol (TC), cholinesterase (ChE), creatine phosphokinase (CPK), and basophil (Ba) as information related to a blood examination of the subject, and in that information output from the seventh learning-completed model is information related to whether the subject is affected with thyrotoxicosis.

In the following description of the seventh embodiment, the same functional configurations and effects as in the first embodiment are denoted by the same reference signs as in the first embodiment and description thereof is omitted, and the difference of the seventh embodiment from the first embodiment will be mainly described.

### 7.1. Hardware configuration

Information related to a hematological examination and acquired by the seventh determination unit 155 includes, for example, creatinine (CREA), total cholesterol (TC), cholinesterase (ChE), creatine phosphokinase (CPK), and basophil (Ba) of a subject and may include other information.

The seventh determination unit 155 executes processing that inputs the age and information related to a hematological examination of the subject, which are acquired as described above, to the seventh learning-completed model and outputs information related to whether the subject is affected with thyrotoxicosis. The seventh learning-completed model is not particularly limited but may be, for example, a learning-completed model generated through machine learning processing based on the above-described learning data 154.

The display unit 157 executes processing that controls display of the above-described information output from the seventh determination unit 155 on the display device. For example, the display unit 157 can control display of the information related to whether the subject is affected with thyrotoxicosis on the display device as the probability that the subject is affected with thyrotoxicosis or the probability that the subject is not affected with thyrotoxicosis.

The learning unit 158 executes processing that generates a learning-completed model through machine learning processing based on the learning data 154. Examples of the machine learning processing include supervised learning and semi-supervised learning.

### 7.2. Learning-completed model

As an example, the seventh learning-completed model was produced by using Prediction One manufactured by Sony Network Communications Inc. The production of the seventh learning-completed model was performed by using learning data of 19335 untreated Graves' disease patients, 3267 untreated painless thyroiditis patients, and 4159 healthy subjects.

Specifically, the learning data was divided at the ratio of 7:3 to prepare a data set of teacher data and test data, and the production of the seventh learning-completed model was performed by using the teacher data. As a result, the seventh learning-completed model that uses creatinine (CREA), total cholesterol (TC), cholinesterase (ChE), creatine phosphokinase (CPK), and basophil (Ba) as prediction factors was obtained.

The ROC curve and AUC of the seventh learning-completed model were calculated by using the above-described test data. The following evaluation result of the seventh learning-completed model was obtained.
AUC 0.951

### 7.3. Operation processing

Operation of the information processing device 100 of the seventh embodiment thus configured will be described below.

At step S202, the learning unit 158 produces teacher data from a data set stored in the learning data 154 and produces, by using the teacher data, the seventh learning-completed model that outputs information related to whether a subject is affected with thyrotoxicosis. For example, the learning unit 158 performs machine learning by using the teacher data with a correct answer label set to information related to whether the subject is affected with thyrotoxicosis.

At step S302, the determination unit 155 inputs information related to a hematological examination result of the subject to the seventh learning-completed model and outputs, from the seventh learning-completed model, information related to whether the subject is affected with thyrotoxicosis. In place of or in addition to this, the determination unit 155 may output, from the seventh learning-completed model, information related to whether thyroid stimulation hormone (TSH) is equal to or less than a normal value.

At step S303, the display unit 156 controls display of the output information on an image output device such as a display. The display control is not limited to a particular method, but for example, may indicate, in percentage, the probability that the subject is affected with thyrotoxicosis and the probability that the subject is not affected with thyrotoxicosis.

### 8. First example

The diagnosis system 1 as the thyroid disease determination device described in each of the first to seventh embodiments may be independently used or may be used in optional combination. For example, combination of the first and second embodiments, combination of the third and seventh embodiments, and combination of the fourth and seventh embodiments are possible.

Some of such examples will be described below.

### 8.1. First example 1

A first example 1 is an example of combination of the first and second embodiments. The information processing device 100 of the example 1 includes the second determination unit that inputs the age of a subject and information related to total protein (TP), creatinine (CREA), neutrophil (Neu), and total bilirubin (T-Bill) to the second learning-completed model from information related to a blood examination of the subject and outputs information related to whether thyroid stimulation hormone (TSH) of the subject is in a normal value range, and the first determination unit that inputs information related to total protein (TP), cholinesterase (ChE), total cholesterol (TC), creatinine (CREA), and creatine phosphokinase (CPK) from the information related to a blood examination of the subject to the first learning-completed model and outputs information related to whether thyroid stimulation hormone (TSH) of the subject is in a range for which medical treatment is needed.

The configuration, operation processing, and the like of the information processing device 100 according to the first example 1 are basically the same as the configuration, operation processing, and the like of the information processing device of the diagnosis system according to each of the first and second embodiments.

In the first example 1, since the first and second embodiments are used in combination, it is possible to evaluate in stages, for example, whether thyroid stimulation hormone (TSH) of a subject is in a range for which medical treatment is needed or in a normal value range.

### 8.2. First example 2

A first example 2 is an example of combination of the third and seventh embodiments. The information processing device 100 of the example 2 includes the seventh determination unit that inputs information related to creatinine (CREA), total cholesterol (TC), cholinesterase (ChE), creatine phosphokinase (CPK), and basophil (Ba) from information related to a blood examination of a subject to the seventh learning-completed model and outputs information related to whether the subject is affected with thyrotoxicosis, and the third determination unit that inputs the age of the subject and information related to creatinine (CREA), total cholesterol (TC), alkaline phosphatase (ALP), and total protein (TP) from the information related to a blood examination of the subject to the third learning-completed model and outputs information related to whether the subject is affected with Graves' disease.

The configuration, operation processing, and the like of the information processing device 100 according to the first example 2 are basically the same as the configuration, operation processing, and the like of the information processing device of the diagnosis system according to each of the third and seventh embodiments.

In the first example 2, since the third and seventh embodiments are used in combination, it is possible to evaluate in stages, for example, whether a subject is affected with thyrotoxicosis and whether the disease is likely to be Graves' disease in a case in which the subject is affected with thyrotoxicosis.

### 8.3. First example 3

A first example 3 is an example of combination of the fourth and seventh embodiments. The information processing device 100 of the example 3 includes the seventh determination unit that inputs information related to creatinine (CREA), total cholesterol (TC), cholinesterase (ChE), creatine phosphokinase (CPK), and basophil (Ba) from information related to a blood examination of a subject to the seventh learning-completed model and outputs information related to whether the subject is affected with thyrotoxicosis, and the fourth determination unit that inputs information related to thyroid hormone FT3, thyroid hormone FT4, FT3/FT4, alkaline phosphatase (ALP), and creatinine (CREA) from the information related to a blood examination of the subject to the fourth learning-completed model and outputs information related to differentiation of whether the subject is affected with Graves' disease or painless thyroiditis.

The configuration, operation processing, and the like of the information processing device 100 according to the first example 3 are basically the same as the configuration, operation processing, and the like of the information processing device of the diagnosis system according to each of the fourth and seventh embodiments.

In the first example 3, since the fourth and seventh embodiments are used in combination, for example, it is possible to evaluate in stages, whether a subject is affected with thyrotoxicosis and whether the disease is likely to be painless thyroiditis in a case in which the subject is affected with thyrotoxicosis.

### 9. Second example

The diagnosis system 1 as the thyroid disease determination device described in each of the first to seventh embodiments may be constituted by a server (information processing device 100) as a visual prognosis determination device and a user terminal 200 that are connected to each other through a communication network such as the Internet to perform communication therebetween.

Figure 4 illustrates a processing sequence of a diagnosis system constituted by the server (information processing device 100) that is a thyroid disease determination device and the user terminal 200. The server 100 is connected to the user terminal 200 through a communication network such as the Internet to perform communication therebetween. The determination unit 155 of the server 100 provides the diagnosis system 1 that inputs biochemical parameters, such as a hematological examination result and age, received from the user terminal 200 to a learning-completed model, outputs each piece of information described in each of the first to seventh embodiments, and transmits the output information to the user terminal 200.

The server 100 is an example of an information processing device on which the entire or part of the diagnosis system of the present invention is implemented, and may have the hardware and functional unit configurations of the above-described information processing device 100. The user terminal 200 may be a typical computer including a display capable of transmitting and receiving information and displaying information.

At step S401, the user terminal 200 transmits information related to biochemical parameters such as a hematological examination result and age to the server 100, and the determination unit 155 of the server 100 acquires the information related to biochemical parameters such as a hematological examination result and age.

At step S402, the determination unit 155 of the server 100 inputs the information received from the user terminal 200 to a learning-completed model and outputs results described in each of the first to seventh embodiments. At step S403, the server 100 transmits the output results to the user terminal 200, and at step S404, the user terminal 200 controls display of the received results on a display device.

Accordingly, any user terminal 200 that can access the server 100 can use the diagnosis system of the present invention, and for example, regional differences of the medical level can be resolved.

The present invention further provides a computer program for causing the information processing device to execute the inputting and outputting of each piece of information to and from a learning-completed model in the above-described first to seventh embodiments, and an information processing method by which the information processing device executes the inputting and outputting of each piece of information to and from a learning-completed model.

### Reference Signs List

1 ... diagnosis system, 100 ... information processing device, 110 ... processor, 120 ... communication interface, 130 ... input-output interface, 140 ... memory, 150 ... storage, 151 ... operating system, 152 ... network communication unit, 153 ... subject data, 154 ... learning data, 155 ... determination unit, 156 ... display unit, 157 ... learning unit, 160 ... communication bus, 200 ... user terminal

## Claims

1. An information processing device comprising a first determination unit that inputs information related to total protein (TP), cholinesterase (ChE), total cholesterol (TC), creatinine (CREA), and creatine phosphokinase (CPK) to a first learning-completed model based on information related to a blood examination of a subject and outputs information related to whether thyroid stimulation hormone (TSH) of the subject is in a range for which medical treatment is needed.

2. An information processing device comprising a second determination unit that inputs information related to total protein (TP), creatinine (CREA), neutrophil (Neu), and total bilirubin (T-Bill) to a second learning-completed model based on an age of a subject and information related to a blood examination of the subject and outputs information related to whether thyroid stimulation hormone (TSH) of the subject is in a normal value range.

3. An information processing device comprising a third determination unit that inputs information related to creatinine (CREA), total cholesterol (TC), alkaline phosphatase (ALP), and total protein (TP) to a third learning-completed model based on an age of a subject and information related to a blood examination of the subject and outputs information related to whether the subject is affected with Graves' disease.

4. An information processing device comprising a fourth determination unit that inputs information related to thyroid hormone FT3, thyroid hormone FT4, FT3/FT4, alkaline phosphatase (ALP), and creatinine (CREA) to a fourth learning-completed model based on information related to a blood examination of a subject and outputs information related to differentiation of whether the subject is affected with Graves' disease or painless thyroiditis.

5. An information processing device comprising a fifth determination unit that inputs information related to alkaline phosphatase (ALP), creatinine (CREA), total protein (TP), γ glutamyl transpeptidase (yGTP), and white blood cells (WBC) to a fifth learning-completed model based on information related to a blood examination of a subject and outputs information related to differentiation of whether the subject is affected with Graves' disease or painless thyroiditis.

6. An information processing device comprising a sixth determination unit that inputs information related to total cholesterol (TC), cholinesterase (ChE), creatinine (CREA), and creatine phosphokinase (CPK) to a sixth learning-completed model based on an age of a subject and information related to a blood examination of the subject and outputs information related to whether the subject is affected with painless thyroiditis.

7. An information processing device comprising a seventh determination unit that inputs information related to creatinine (CREA), total cholesterol (TC), cholinesterase (ChE), creatine phosphokinase (CPK), and basophil (Ba) to a seventh learning-completed model based on information related to a blood examination of a subject and outputs information related to whether the subject is affected with thyrotoxicosis.
